(19) Europäisches Patentamt
European Patent Office
Office européen des brevets

(11) Numéro de publication : **0 410 848 B1**

(12) **FASCICULE DE BREVET EUROPEEN**

(45) Date de publication du fascicule du brevet :
**29.12.93 Bulletin 93/52**

(51) Int. Cl.$^5$ : **A61K 9/127,** A61K 39/35

(21) Numéro de dépôt : **90402065.8**

(22) Date de dépôt : **18.07.90**

(54) **Procédé pour combiner un mélange de substances hétérogènes à des liposomes.**

Le dossier contient des informations techniques présentées postérieurement au dépôt de la demande et ne figurant pas dans le présent fascicule.

(30) Priorité : **27.07.89 FR 8910129**

(43) Date de publication de la demande :
**30.01.91 Bulletin 91/05**

(45) Mention de la délivrance du brevet :
**29.12.93 Bulletin 93/52**

(84) Etats contractants désignés :
**AT BE CH DE DK ES GB GR IT LI LU NL SE**

(56) Documents cités :
**EP-A- 0 158 880
WO-A-87/01586
WO-A-89/10753
DE-A- 3 433 609
FR-A- 2 276 062**

(73) Titulaire : **LABORATOIRE DES STALLERGENES
7 allée des Platanes
F-94250 Fresnes (FR)**

(72) Inventeur : **Millet-Genin, Isabelle
69 chemin Schoelcher
F-78370 Plaisir (FR)**
Inventeur : **Puisieux, Francis
66 rue de Strasbourg
F-94700 Maisons Alfort (FR)**
Inventeur : **Thao, Tran Xuan
11 rue des Prés Hauts
F-92290 Chatenay Malabry (FR)**
Inventeur : **Roblot-Treupel, Liliane
3 Place de l'Eglise
F-94320 Thiais (FR)**

(74) Mandataire : **Bernasconi, Jean et al
Cabinet Michel Lemoine 13 Boulevard des Batignolles
F-75008 Paris (FR)**

## Description

L'invention est relative à un procédé pour combiner à des liposomes des substances hétérogènes contenues dans un mélange, notamment des protides ou des acides nucléiques (ou leurs dérivés nucléosides, nucléotides), et en particulier des substances allergéniques présentes dans une préparation allergénique, par adsorption à la surface des, et/ou incorporation (encapsulation) dans les liposomes, afin notamment de produire des compositions d'immunisation, en particulier des compositions de désensibilisation de patients allergiques, ou encore des bioréactifs.

Par préparation allergénique, on entend notamment des préparations comprenant des allergènes et/ou des extraits allergéniques divers, par exemple des pollens, des acariens, des phanères ou des extraits de ceux-ci, ou encore toutes autres substances ou protéines allergéniques animales ou végétales.

Les liposomes sont des vésicules formées d'une ou plusieurs bicouches de phospholipides et d'un ou plusieurs compartiments aqueux. Ils sont donc unilamellaires ou multilamellaires.

Les liposomes présentent un certain nombre d'atouts dans le domaine de l'administration thérapeutique:
- Ils ne sont pas ou peu toxiques car ils peuvent être constitués de lipides naturels.
- Quand ils sont administrés par voie sous-cutanée, ils favorisent le drainage du principe actif vers le système lymphatique. Ceci est doublement avantageux car, en empruntant cette voie, les liposomes ne sont pas en contact avec les éléments figurés du plasma. De plus, ils rencontrent rapidement les éléments du système immunitaire car ceux-ci sont principalement localisés dans la circulation lymphatique.
- L'utilisation de liposomes multilamellaires permet d'obtenir aisément un système à libération prolongée in vivo, donc un effet retard, ce qui est important dans le domaine en question. En multipliant le nombre de bicouches, donc le nombre de compartiments aqueux, on augmente la durée d'action.

De nombreux travaux utilisant des liposomes comme adjuvants immunologiques dans des vaccins ont donc été menés (toxine diphtérique, toxine tétanique, toxine cholérique, venins de serpent, glycoprotéine rabique, adénovirus, hépatite, sous-unités de virus influenza, Plasmodium yoeli).

Il a également déjà été réalisé des liposomes contenant des allergènes :
- G.A. Stewart et A.S. McWilliam ( Immune responses to liposome entrapped mite allergens - Mite allergy, a world-wide problem, Septembre 1987, The U.C.B. Institute of Allergy) ont incorporé des allergènes d'acariens dans trois types de liposomes, des liposomes unilamellaires ("SUV" pour "small unilamellar vesicles"), des liposomes multilamellaires ("MLV" pour "multilamellar vesicles"), des liposomes obtenus par un procédé comprenant une étape d'évaporation du solvant des liposomes en présence des allergènes ("REV" pour "reverse evaporation phase vesicles"). Ils ont montré que les liposomes avaient des affinités différentes vis-à-vis des allergènes.
- G. Jederström ( Resume of a Licentiate Thesis from the Faculté of Pharmacy, Uppsala University- Pharmaceutical Research and Development, Pharmacia AB, Box 181, S-751 82 UPPSALA, Sweden ) a montré l'activité immunogène in vivo de liposomes contenant du pollen.
- A.S Mc WILLIAM et G.A. STEWART (Journal of Immunological Methods,121, (1989),53-60) ont décrit des liposomes ("MLV", "SUV" et "REV") contenant un extrait allergénique d'acariens (Dermatophagoides pteronyssinus) en vue d'une immunothérapie. La technique de préparation des liposomes multilamellaires passe par une phase de chauffage à 50°C pendant 20 mn, ce qui paraît bien élevé et risque de dénaturer l'extrait allergénique. L'une des préparations utilisant les "MLV" a permis d'incorporer, mais avec un faible rendement, le spectre d'allergènes de l'extrait étudié. La recherche d'une augmentation du rendement a conduit à des solutions excluant l'incorporation de certains constituants de l'extrait.

Les liposomes sont généralement constitués de phospholipides et de cholestérol. Pour augmenter le rendement de l'encapsulation, on peut y associer un lipide ionique chargé de façon positive (+) ou négative (-) selon l'allergène considéré. Cette amélioration de l'encapsulation résulte à la fois d'une augmentation du volume des compartiments aqueux par répulsion des charges entre bicouches et par attraction entre le lipide ionique et l'allergène de charge opposée.

Ainsi, le brevet européen EP-A-0.158.880 propose de conférer une polarité donnée aux liposomes pour améliorer l'incorporation de molécules polaires.

On peut donc incorporer individuellement au liposome une grande variété de composés hydrosolubles, les liposomes ayant une affinité plus ou moins grande selon les allergènes considérés.

Par contre, la combinaison, à des liposomes, de mélanges de substances diverses, ayant notamment des caractéristiques physico-chimiques différentes, et notamment d'allergènes, n'a pas permis d'obtenir, avec un rendement convenable, une adsorption ou encapsulation homogène des divers constituants du mélange. Or, en allergologie notamment, on rencontre souvent des mélanges contenant un large éventail de substances allergéniques que l'on ne connaît pas toujours de manière précise, éventail dont il faudrait au minimum reconstituer le profil dans une composition retard de désensibilisation.

L'invention a donc pour objectif de fournir un procédé pour combiner à des liposomes chargés de façon positive (+) ou négative (-) des substances hétérogènes contenues dans un mélange, par adsorption à la surface des, et/ou incorporation dans les, liposomes, qui permette la fixation en grandes quantités de toute substance selon l'invention contenue dans le mélange, tout en conservant sensiblement le profil de distribution initial dudit mélange.

Un autre objectif de l'invention est de fournir un procédé qui s'applique à la constitution de tous les types de liposomes (notamment "MLV", "SUV", "REV").

Un autre objectif encore de l'invention est de fournir un tel procédé qui soit facile et rapide à mettre en oeuvre.

Un autre objectif de l'invention est de fournir des compositions à effet retard _in vivo_, qui aient des qualités équivalentes à celles des mélanges de substances de départ.

La présente invention a pour objet un procédé du type décrit au début dans lequel les liposomes comportent du cholestérol, un phospholipide et/ou au moins un lipide ionique qui confère au liposome une charge positive ou négative. Ce procédé est caractérisé en ce qu'on met en présence du liposome ou de ses constituants le mélange de substances hétérogènes, le pH de l'ensemble étant supérieur ou inférieur au point isoélectrique pi des substances contenues dans le mélange, selon que le lipide ionique est chargé de façon positive ou négative respectivement.

Le choix de la charge + ou - conférée au liposome dépend notamment d'un critère essentiel, à savoir que les substances devront pouvoir supporter un pH alcalin ou un pH acide selon le cas.

Si le mélange est mis en présence des constituants du liposome, on obtient une adsorption de substances à la surface des liposomes ainsi qu'une incorporation de substances dans ceux-ci.

Si le mélange est mis en présence des liposomes déjà formés, on obtient une simple adsorption des substances à la surface des liposomes.

Dans le premier cas, on pourra, de manière usuelle, jouer notamment sur le nombre de bicouches ou sur la composition du liposome pour augmenter l'effet retard de la composition qui en résultera.

De manière avantageuse, le pH de l'ensemble formé par les liposomes, ou leurs constituants, et le mélange de substances hétérogènes est supérieur ou inférieur au pi de la substance dont le pi est le plus fort ou le plus faible respectivement.

Le lipide ionique peut être notamment la stéarylamine ou SA (chargée +) ou le dicétylphosphate ou DCP (chargé -).

Le phospholipide peut être notamment la phosphatidylcholine de soja (PCS) ou le dipalmitoylphosphatidylcholine (DPPC).

Dans un mode de réalisation particulier de l'invention, le lipide ionique peut même être un phospholipide chargé, tel que la phosphatidylsérine notamment.

La formation des liposomes, dans les conditions de pH selon l'invention, peut notamment être réalisée selon les procédés connus qui conduisent aux trois types de liposomes cités ci-dessus (G.A. Stewart et al. cité ci-dessus):

- Mélanger la solution aqueuse contenant les substances hétérogènes aux constituants des liposomes (contenus dans un solvant organique), puis effectuer une sonication. Eliminer les solvants organiques. On obtient des liposomes "REV" par évaporation du solvant en présence des substances hétérogènes.
- Dissoudre les constituants des liposomes dans un solvant volatile, évaporer dans un évaporateur rotatif; les constituants forment alors un film au contact de la paroi de l'évaporateur; ajouter la solution aqueuse de substances hétérogènes pour reprendre le film. Il s'agit à la base de la méthode de Bangham (BURI p., PUISIEUX F., DOELKER F., BENOIT J.P., Vecteurs micro et nanoparticulaires. Formes pharmaceutiques nouvelles. Aspects technologiques, biopharmaceutique et médical. Ed. Lavoisier, Tec et Doc, 1985, 463-575). On obtient des liposomes multilamellaires "MLV".
- Sous agitation, ajouter lentement les constituants lipidiques (en solution dans un solvant) à la solution de substances hétérogènes. On obtient des liposomes unilamellaires "SUV".

Bien entendu, dans le cas où le procédé selon l'invention consiste dans la mise en présence du mélange de substances hétérogènes et de liposomes déjà constitués, la solution de substances hétérogènes est remplacée dans les étapes ci-dessus par de l'eau distillée ou un tampon, afin d'obtenir des liposomes "blancs".

L'invention a également pour objet des compositions obtenues avec le procédé selon l'invention à partir de préparations allergéniques, pour la désensibilisation de patients allergiques.

L'invention a encore pour objet des compositions obtenues avec ledit procédé pour la constitution de bioréactifs in vitro dans les domaines de l'immunologie ou de l'allergologie notamment. Il peut s'agir par exemple de liposomes contenant en sus, de façon connue en soi, un révélateur, notamment un indicateur coloré. On peut aussi envisager des réactions d'agglutination ou encore l'utilisation d'enzymes.

Un exemple de composition allergénique avec liposome "MLV", pour la désensibilisation ou pour la constitution de bioréactifs, est le suivant :

- phosphatidylcholine de soja          200 mg
- cholestérol          30 mg
- dicétylphosphate          10 mg
- $\alpha$ - tocophérol          10 mg
- tampon ou diluant physiologique phénolé qsp 10 ml
- préparation allergénique dosée.

L'invention a encore pour objet des compositions obtenues avec ledit procédé à partir de molécules immunogènes, notamment des séquences peptidiques ou nucléotidiques immunogènes, pour la constitution de vaccins (hépatite, grippe, polio, etc.)

La déposante a découvert en outre qu'il était possible de lyophiliser les compositions dans lesquelles des substances hétérogènes sont incorporées sans addition d'agents cryoprotecteurs. La présence desdites substances hétérogènes suffirait à stabiliser les liposomes.

Dans le cas où les substances hétérogènes sont uniquement adsorbées, il peut être utile d'ajouter de la manière habituelle un agent cryoprotecteur.

L'invention a donc pour objet des compositions du type décrit plus haut qui ont été lyophilisées avec ou sans agent cryoprotecteur.

L'invention va être maintenant décrite plus en détail à l'aide d'un exemple non limitatif, concernant la combinaison de substances allergéniques selon l'invention à des liposomes multilamellaires du type "MLV".

Les constituants des liposomes utilisés dans cet exemple sont les suivants:

1 - pour les phospholipides:
- la phosphatidylcholine de soja, PCS, épikuron 200, de poids moléculaire 780 (Lucas Meyer France SA, Saint Maur Les Fossés),
- la dipalmitoylphosphatidylcholine, DPPC, de poids moléculaire 734 (Sigma SARL, La Verpillère, France).

2 - pour les lipides ioniques:
- la stéarylamine, SA, qui apporte une charge positive aux liposomes (Sigma Chimie SARL), poids moléculaire = 269,5,
- le dicétylphosphate, DCP, qui apporte une charge négative (Sigma Chimie SARL) ; poids moléculaire = 549,9

3 - du cholestérol, CH (Sigma Chimie SARL)

4 - éventuellement un antioxydant, par exemple l'$\alpha$-tocophérol, $\alpha$T, pour empêcher l'oxydation du phospholipide.

La préparation des liposomes peut être réalisée par la méthode de Bangham déjà citée:
- Dissolution des lipides dans du chloroforme à une concentration de 20mg de phospholipides par millilitre.
- Evaporation sous vide dans un évaporateur rotatif à 35°C pour les liposomes à base de PCS, et à 50°C pour les liposomes à base de DPPC. Il se forme un film sur les parois.
- Reprise du film par une solution aqueuse, à une température supérieure à la température de transition de phase du mélange lipidique. Dans le cas de la PCS, on travaille à la température ambiante, dans celui de la DPPC, on travaille à 40°C.

Par simple agitation, les lipides se décollent de la paroi du ballon évaporateur et forment une suspension laiteuse de liposomes multilamellaires. Les phospholipides adoptent spontanément l'organisation en bicouches quand ils sont dispersés dans un excès d'eau. La suspension de liposomes obtenue est laissée à gonfler à 4°C.

L'incorporation des allergènes peut se faire de deux façons:
- Reprise du film ci-dessus par la solution d'allergènes comme solution aqueuse. Ceux-ci vont se retrouver entre les bicouches et à la surface des liposomes. C'est cette méthode qui est décrite par Bangham. On arrive à des liposomes de type B.
- Reprise du film par un tampon ou par de l'eau distillée. On obtient alors des liposomes blancs. Dans un second temps, on procède au mélange de ces liposomes avec une solution d'allergènes. Ceux-ci vont se retrouver exclusivement à la surface des liposomes et ces liposomes sont appelés liposomes de type A.

Dans un mode de réalisation avantageux, le procédé selon l'invention, appliqué à des liposomes chargés positivement (contenant de la SA), consiste à conférer à la solution aqueuse d'allergènes, à l'aide d'HCl 0,1N, un pH tel que, après mise en présence de ladite solution et des liposomes ou de leurs constituants selon le cas, le mélange obtenu présente finalement un pH supérieur au pi de l'allergène (les allergènes ont en général un pi situé entre environ 4 et environ 6) dont le pi est le plus fort.

Dans le cas de liposomes chargés négativement (contenant du DCP), le mélange obtenu devra présenter un pH inférieur au pi de l'allergène dont le pi est le plus faible.

On peut bien sûr envisager d'autres façons d'amener le pH du mélange à la valeur désirée. On peut par exemple ajuster le pH après la mise en présence des allergènes et des liposomes ou de leurs constituants.

L'exemple qui suit porte sur les allergènes suivants :
- des pollens (cinq graminées)
- des acariens (Dermatophagoides ptéronyssinus ou DPtero, et Dermatophagoides farinae)
- des phanères (poils de chat et poils de chien)
- un extrait du pollen d'une graminée, la Dactyle (il s'agit en fait d'un mélange de plusieurs protéines, donnant 20 bandes à l'électrofocalisation).

La formule retenue pour préparer les liposomes est la suivante : PCS/Ch/lipide ionique SA ou DCP/$\alpha$T, pour un rapport molaire de 7/2/0,5/1.

Dans un premier temps, on a fixé les allergènes aux liposomes en suivant seulement la méthode de Bangham.

Les résultats (rendements d'incorporation) obtenus sont regroupés dans le tableau I qui suit. Il concerne des liposomes de type B.

## Tableau I

|  | PCS/Ch/SA | PCS/Ch/DCP |
|---|---|---|
| Pollens | 10 à 20 % | 50 % |
| Acariens | 10 à 15 % | 60 % |
| Phanères | 50 à 60 % | 10 à 20 % |

Ce tableau fait ressortir les différences d'affinité des liposomes vis-à-vis des différents allergènes.

On a ensuite appliqué le procédé selon l'invention à la construction de liposomes de type B contenant des allergènes de poils de chat.

Le tableau II qui suit réunit les résultats obtenus dans le cas où le pH du mélange liposomes-allergènes est supérieur au point isoélectrique (pi) des allergènes (cas que l'on rencontre en suivant la méthode de Bangham) et dans le cas où l'on réduit le pH, par exemple avec une solution d'HCl 0,1N, jusqu'à une valeur finale inférieure au pi des allergènes, le lipide ionique utilisé étant le DCP (charge négative).

## Tableau II

|  | pH | IR |
|---|---|---|
| Solution d'allergènes | 7,64 | 54 |
| Liposomes blancs avec DCP | 3,17 | 0 |
| Liposomes avec DCP + solution d'allergènes à pH 7,64 (2 essais) | 1. 7,59<br>2. 7,81 | 5,5<br>3,5 |
| Liposomes avec DCP + solution d'allergènes à pH 3,92 (2 essais) | 1. 3,57<br>2. 3,55 | 44<br>43 |

IR : indice de réactivité interne.

Cet indice est obtenu par dosage radio-immunologique, par la méthode du RAST-inhibition (Radio Allergo Sorbent Test) qui mesure l'activité immunologique des allergènes.

L'indice de réactivité cutanée 100 IR est défini in vivo sur une population de 30 sujets sensibles à l'allergène de telle sorte qu'après scarification par piqûre sur la face interne de l'avant-bras, la surface moyenne de la papule produite soit de 30 mm$^2$, soit un diamètre d'environ 6 mm.

Dans le cas d'allergènes se trouvant encapsulés dans les liposomes, il faut au préalable séparer les allergènes de leur support. Pour cela, on utilise la méthode proposée par JULIANO R.L. et LIN G. (The interaction of plasma proteins with liposomes : protein binding and effects on the dotting and complement systems - Liposomes and Immunology, 1980, Tom I Six by Elsevier). Ils utilisent de l'éthanol pour solubiliser les lipides constituant les liposomes et pour isoler les protéines par précipitation.

On centrifuge les liposomes, puis:
- on dose l'activité résiduelle du surnageant par RAST-inhibition;
- on met le culot de liposomes en contact avec 9 volumes d'éthanol absolu pendant 1 heure à 0°C, on centrifuge 15 mm à 5000 tr/mn, on récupère le culot de protéines obtenu et on le dissout dans du PBS; on dose alors l'activité par RAST-inhibition.

Le tableau II ci-dessus démontre bien l'efficacité du procédé selon l'invention. En se plaçant à un pH<pi dans le cas d'un liposome chargé négativement, on obtient un rendement d'adsorption et d'incorporation des allergènes de poils de chat supérieur à 50 %.

On a obtenu des résultats équivalents avec des allergènes de Dactyle et de DPtero.

La lyophilisation des allergènes ne pose pas de problèmes particuliers puisqu'elle est utilisée actuellement pour assurer la conservation des extraits allergéniques.

La lyophilisation des liposomes combinés à des allergènes doit répondre aux critères suivants :
- conservation de l'intégrité structurale des liposomes,
- taux d'adsorption et d'incorporation des allergènes constant,
- dispersion facile des liposomes lyophilisés après reprise du lyophilisat par une phase aqueuses appropriée.

Les conditions de lyophilisation sont une congélation à -30°C puis une sublimation pendant 20 heures. On a utilisé un lyophilisateur Alpha I-5 (Bioblock Scientific, Paris).

Tous les critères ont été respectés, quelle que soit la composition des liposomes.

La déposante a découvert que les allergènes encapsulés jouent probablement le rôle de cryoprotecteur.

On va maintenant donner un exemple de composition allergénique selon l'invention, pour des liposomes du type MLV :

- phosphatidylcholine de soja     200 mg
- cholestérol     30 mg
- dicétylphosphate     10 mg
- α - tocophérol     10 mg
- solution qsp 10 ml
- extrait de Dactyle dosé en I.R.

Comme solution, on peut utiliser tout diluant physiologique phénolé ainsi que tout tampon physiologique phénolé.

Cette composition peut être utilisée pour la désensibilisation de patients allergiques ou pour la constitution de bioréactifs.

**Revendications**

1. Procédé pour combiner à des liposomes des substances hétérogènes contenues dans un mélange, notamment des substances allergéniques, telles que des allergènes et/ou des extraits allergéniques, contenues dans une préparation allergénique, par adsorption à la surface des, et/ou incorporation dans les, liposomes, lesquels comportent du cholestérol, un phospholipide et/ou au moins un lipide ionique qui confère au liposome une charge positive ou négative, caractérisé en ce qu'on met en présence du liposome ou de ses constituants le mélange de substances hétérogènes, le pH de l'ensemble étant supérieur ou inférieur au point isolélectrique pi des substances contenues dans le mélange, selon que le lipide ionique est chargé de façon positive ou négative respectivement.

2. Procédé selon la revendication 1, caractérisé en ce que le pH de l'ensemble formé par les liposomes ou les constituants des liposomes et le mélange de substances hétérogènes est supérieur ou inférieur au pi de la substance dont le pi est le plus fort ou le plus faible respectivement.

3. Procédé selon l'une des revendications 1 et 2, caractérisé en ce que l'on confère à la solution aqueuse de substances hétérogènes, à savoir des allergènes, un pH tel que, après mise en présence de ladite solution et des liposomes ou de leurs constituants, le mélange obtenu présente finalement un pH supérieur au pi de l'allergène, si les liposomes sont chargés positivement, ou inférieur au pi de l'allergène si les liposomes sont chargés négativement.

4. Procédé selon la revendication 3, caractérisé en ce que l'on modifie le pH en utilisant une solution d'HCl 0,1 N.

5. Procédé selon l'une des revendications 1 à 4, caractérisé en ce que le lipide ionique est la stéarylamine.

6. Procédé selon l'une des revendications 1 à 4, caractérisé en ce que le lipide ionique est le dicétylphosphate.

7. Procédé selon l'une quelconque des revendications 1 à 6, caractérisé en ce que le phospholipide est la phosphatidylcholine de soja ou le dipalmitoylphosphatidylcholine.

8. Procédé selon l'une des revendications 1 à 4, caractérisé en ce que le lipide ionique est un phospholipide.

9. Procédé selon la revendication 8, caractérisé en ce que le phospholipide est la phosphatidylsérine.

10. Procédé selon l'une quelconque des revendications 1 à 9, caractérisé en ce que le liposome obtenu est un liposome multilamellaire du type "MLV".

11. Procédé selon l'une quelconque des revendications 1 à 9 caractérisé en ce que le liposome est un liposome unilamellaire du type "SUV".

12. Procédé selon l'une quelconque des revendications 1 à 9, caractérisé en ce que le liposome obtenu est du type "REV".

13. Composition obtenue avec le procédé selon l'une des revendications 1 à 12, à partir de préparations allergéniques, pour la désensibilisation de patients allergiques.

14. Composition obtenue avec le procédé selon l'une des revendications 1 à 12, pour la constitution de bioréactifs.

15. Composition selon la revendication 13 ou 14, caractérisé en ce qu'elle comprend des liposomes "MLV" constitués par 200 mg de phospatidylcholine, de 30 mg de cholestérol, de 10 mg de dicétylphosphate et de 10 mg d'$\alpha$-tocophérol, une quantité suffisante de tampon ou de diluant physiologique phénolé pour obtenir un volume de 10 ml et une préparation allergénique dosée.

16. Composition obtenue avec le procécé selon l'une des revendications 1 à 12, à partir de molécules immunogènes, pour la constitution de vaccins.

17. Composition selon l'une quelconque des revendications 13 à 16, caractérisé en ce qu'elle est lyophilisée, la lyophilisation ayant eu lieu avec ou sans adjonction de cryoprotecteur.


## Patentansprüche

1. Verfahren zum Vereinigen von in einer Mischung enthaltenen heterogenen Substanzen, insbesondere in einem allergenen Präparat enthaltenen allergenen Substanzen, wie beispielsweise Allergenen und/oder allergenen Extrakten, mit Liposomen durch Adsorption auf der Oberfläche der und/oder Inkorporierung in die Liposomen, die Cholesterol, ein Phospholipid und/oder mindestens ein ionisches Lipid, das dem Liposom eine positive oder negative Ladung verleiht, umfassen, dadurch gekennzeichnet, daß man die Mischung von heterogenen Substanzen mit dem Liposom oder dessen Bestandteilen zusammenbringt, wobei der pH des Ganzen je nachdem, ob das ionische Lipid auf positive oder negative Weise geladen ist, oberhalb bzw. unterhalb des isoelektrischen Punkts pi der in der Mischung enthaltenen Substanzen liegt.

2. Verfahren nach Anspruch 1, dadurch gekennzeichnet, daß der pH des Ganzen, das durch die Liposomen oder die Bestandteile der Liposomen und die Mischung von heterogenen Substanzen gebildet wird, oberhalb oder unterhalb des pi der Substanz liegt, deren pi der höchste bzw. der niedrigste ist.

3. Verfahren nach einem der Ansprüche 1 und 2, dadurch gekennzeichnet, daß man der wäßrigen Lösung von heterogenen Substanzen, nämlich der Allergene, einen derartigen pH verleiht, daß nach Zusammenbringen des besagten Lösung und der Liposomen oder deren Bestandteilen die erhaltene Mischung am Ende einen pH oberhalb des pi des Allergens, wenn die Liposomen positiv geladen sind, oder unterhalb des pi des Allergens, wenn die Liposomen negativ geladen sind, aufweist.

4. Verfahren nach Anspruch 3, dadurch gekennzeichnet, daß man den pH durch Verwendung einer HCl-Lösung ändert.

5. Verfahren nach einem der Ansprüche 1 bis 4, dadurch gekennzeichnet, daß das ionische Lipid Stearylamin ist.

6. Verfahren nach einem der Ansprüche 1 bis 4, dadurch gekennzeichnet, daß das ionische Lipid Dicetylphosphat ist.

7. Verfahren nach irgendeinem der Ansprüche 1 bis 6, dadurch gekennzeichnet, daß das Phospholipid Soja-Phosphatidylcholin oder Dipalmitoylphosphatidylcholin ist.

8. Verfahren nach einem der Ansprüche 1 bis 4, dadurch gekennzeichnet, daß das ionische Lipid ein Phospholipid ist.

9. Verfahren nach Anspruch 8, dadurch gekennzeichnet, daß das Phospholipid Phosphatidylserin ist.

10. Verfahren nach irgendeinem der Ansprüche 1 bis 9, dadurch gekennzeichnet, daß das erhaltene Liposom ein multilamellares Liposom vom "MLV"-Typ ist.

11. Verfahren nach irgendeinem der Ansprüche 1 bis 9, dadurch gekennzeichnet, daß das Liposom ein unilamellares Liposom vom "SUV"-Typ ist.

**12.** Verfahren nach irgendeinem der Ansprüche 1 bis 9, dadurch gekennzeichnet, daß das erhaltene Liposom vom "REV"-Typ ist.

**13.** Zusammensetzung, erhalten nach dem Verfahren gemäß einem der Ansprüche 1 bis 12 ausgehend von allergenen Präparaten, für die Desensibilisierung von allergischen Patienten.

**14.** Zusammensetzung, erhalten nach dem Verfahren gemäß einem der Ansprüche 1 bis 12, für die Bildung von Bioreagenzien.

**15.** Zusammensetzung nach Anspruch 13 oder 14, dadurch gekennzeichnet, daß sie "MLV"-Liposomen, aufgebaut aus 200 mg Phosphatidylcholin, 30 mg Cholesterol, 10 mg Dicetylphosphat und 10 mg $\alpha$-Tocopherol, eine ausreichende Menge an phenolisiertem Puffer oder physiologischem Verdünnungsmittel, um ein Volumen von 10 ml zu erhalten, und ein abgemessenes allergenes Präparat umfaßt.

**16.** Zusammensetzung, erhalten nach dem Verfahren gemäß einem der Ansprüche 1 bis 12 ausgehend von immunogenen Molekülen, für die Bildung von Impfstoffen.

**17.** Zusammensetzung nach irgendeinem der Ansprüche 13 bis 16, dadurch gekennzeichnet, daß sie lyophilisiert ist, wobei die Lyophilisierung mit oder ohne Zugabe von Kryoschutzmittel stattgefunden hat.

## Claims

**1.** A method for combining with liposomes, heterogeneous substances contained in a mixture, in particular allergenic substances, such as allergens and/or allergenic extracts, contained in an allergenic preparation, by adsorption on the surface of, and/or incorporation in, liposomes which comprise cholesterol, a phospholipid and/or at least one ionic lipid which gives the liposome a positive or negative charge, characterized in that the liposome or its constituents are combined with the mixture of heterogeneous substances, the pH of the whole being higher or lower than the isoelectric point ip of the substances contained in the mixture, depending on wether the ionic lipid is positively or negatively charged respectively.

**2.** A method according to claim 1, characterized in that the pH of the whole formed by the liposomes or liposome constituents and the mixture of the heterogeneous substances is higher or lower than the ip of the substance whose ip is the strongest or weakest respectively.

**3.** A method according to anyone of claims 1 and 2, characterized in that it is given the aqueous solution of heterogeneous substances, that are allergens, a pH such as, after said solution and liposomes or its constituents were combined, the resulting mixture has finally a pH higher than the isoelectric point ip of the allergen if the liposomes are positively charged, or lower than the isoelectric point ip of the allergen if the liposomes are negatively charged.

**4.** A method according to claim 3, characterized in that the pH is changed by using an HCl solution.

**5.** A method according to anyone of claims 1 to 4, characterized in that the ionic lipid is the stearylamine.

**6.** A method according to anyone of claims 1 to 4, characterized in that the ionic lipid is the dicetylphosphate.

**7.** A method according to anyone of claims 1 to 6, characterized in that the phospholipid is the phosphatidylcholine of soya or the dipalmitoylphosphatidylcholine.

**8.** A method according to anyone of claims 1 to 4, characterized in that the ionic lipid is a phospholipid.

**9.** A method according to claim 8, characterized in that the phospholipid is the phosphatidylserine.

**10.** A method according to anyone of claims 1 to 9, characterized in that the liposome obtained is a "MLV"-type multilamellar liposome.

**11.** A method according to anyone of claims 1 to 9, characterized in that the liposome is a "SUV"-type unilamellar liposome.

12. A method according to anyone of claims 1 to 9, characterized in that the liposome obtained is of the "REV"-type.

13. A composition obtained with the method according to anyone of claims 1 to 12, from allergenic preparations, for desensitizing allergic patients.

14. A composition obtained with the method according to anyone of claims 1 to 12, for forming bioreactives.

15. A composition according to claim 13 or 14, characterized in that it comprises "MLV"-liposomes consisting of 200 mg of phosphatidylcholine, 30 mg of cholesterol, 10 mg of dicetylphosphate and 10 mg of $\alpha$-tocopherol, a sufficient quantity of physiological phenol diluent or buffer to obtain a volume of 10 ml and a proportioned allergenic preparation.

16. A composition obtained with the method according to one of the claims 1 to 12, from immunological molecules, for making up vaccines.

17. A composition according to anyone of the claims 13 to 16, characterized in that it is freeze-dried, the freeze-drying having taken place with or without the addition of a cryoprotector.